# EUROPÄISCHE PATENTSCHRIFT

(11) **EP 1 349 609 B1**
(45) Veröffentlichungstag und Bekanntmachung des Hinweises auf die Patenterteilung: **03.10.2007**
(21) Anmeldenummer: 02755000.3
(22) Anmeldetag: 19.08.2002
(51) Int. Cl.: A61N 1/30

(54) **IONTOPHORESE-KALTKONTAKTELEKTRODENSYSTEM**
COLD-CONTACT ELECTRODE SYSTEM FOR IONTOPHORESIS
ELECTRODE A CONTACT FROID IONTOPHORETIQUE

(30) Priorität: 23.08.2001 DE 10141254
(43) Veröffentlichungstag der Anmeldung: 08.10.2003
(60) Teilanmeldung: 06025837.3
(73) Patentinhaber: Edel, Susann, 82031 Grünwald (DE)
(72) Erfinder: Edel, Susann, 82031 Grünwald (DE); Winkler, Rosemarie, 82377 Penzberg (DE)
(74) Vertreter: Banzer, Hans-Jörg
(86) Internationale Anmeldenummer: PCT/EP2002/009251
(87) Internationale Veröffentlichungsnummer: WO 2003/018116

(56) Entgegenhaltungen:
- WO-A-01/13989
- US-A- 5 169 384
- US-A- 5 885 211
- US-A- 6 148 232

## Beschreibung

Die vorliegende Erfindung betrifft ein Iontophorese-Kaltkontaktelektrodensystem mit einer definiert gekühlten Elektrodenoberfläche für den gleichzeitigen Vorgang der Iontophorese-vorbehandlung durch das Abradieren der gekühlten, trockenen, verhornten schlecht stromleitenden Hautschichten und der optimierten Iontophorese-Behandlung durch das verbesserte Eindringen des Iontophorese-Stromes in die gekühlte und besser stromleitende Hautmembran zum iontophoretischen Einbringen von kosmetischen oder medizinischen Wirkstoffen über die menschliche Hautmembran in das darunterliegende Körpergewebe.

Die seit vielen Jahren angewendete Iontophorese ist eine Methode, um elektrisch geladene, also ionisierte kosmetische oder medizinische Wirkstoffe über die Oberhaut in das darunterliegende Körpergewebe einzubringen. Dabei wird die elektrisch geladene Wirkstoffmischung entweder direkt oder mittelbar in einem Wirkstoffträger auf die Anwenderelektrode, genannt Kontaktelektrode, gebracht, welche dann an der gewünschten Stelle mit der Haut kontaktiert wird, wobei nur die Wirkstoffmischung oder der Wirkstoffträger nicht aber die Elektrode selbst mit der Haut in Berührung kommen darf. Die Gegenelektrode, genannt Groundelektrode, wird an einer anderen Hautpartie kontaktiert. Zwischen den beiden Elektroden wird nun eine elektrische Spannung angelegt, welche ein elektrisches Feld erzeugt, so daß ein sogenannter Iontophoresestrom durch den Körper fließt, der die Wirkstoffmolekülionen durch die Haut transportiert. Die elektrische Ladung des Wirkstoffes bestimmt dabei die Polarität an den beiden Elektroden. Beispielsweise ist bei positiver Wirkstoff-Ladung die Kontaktelektrode positiv und die Groundelektrode negativ gepolt, so dass die positiv geladenen Wirkstoffmoleküle von der Kontaktelektrode weg in Richtung der Groundelektrode durch die Oberhaut in das darunterliegende Gewebe transportiert werden.

Wie bereits in der Patentschrift DE 694 25 728 T2 erwähnt, kann durch Auflegen von Eis- oder Wärmebeuteln die elektrische Leitfähigkeit der Haut beeinflußt werden. Das Auflegen der Kühl- oder Wärmebeutel wäre jedoch nur jeweils eine Vor- oder Nachkonditionierung mit kurzzeitiger Wirkung auf die Haut und dem zusätzlichen Nachteil, dass diese bisherige Technik ein schnelles Umschalten auf Heizen oder Kühlen sowie einen dauerhaften Einfluss auf die Hautoberflächentemperatur unmittelbar bei Ablauf der Behandlung nicht ermöglicht.

Seit vielen Jahren ist auch bekannt, dass eine Verbesserung der Iontophorese bzw. des Eindringverhaltens bestimmter Wirkstoffe in die Haut durch starkes Vorkühlen der Haut erzielt wird. Zum einen dadurch, dass die trockenen, verhornten, elektrisch schlecht leitfähigen Hautschichten nach einer Kühlung besser abradiert werden können und zum anderen dadurch, dass der Iontophoresestrom in die gekühlte und dadurch elektrisch leitfähigere Hautmembran besser eindringt. Erzielt wird die Vorkühlung der Haut bisher durch Eispackungen oder andere Kältepackungen, die vor der eigentlichen Iontophoresebehandlung auf die jeweilige Hautpartie aufgelegt werden. Der Nachteil dieser Methoden ist, dass mehrere Vorgänge durchgeführt werden müssen und dass die Vorkühlung der Haut schnell nachlässt und somit der erzielbare Kühleffekt während der lontophorese kaum noch anhält und auch kein Abrasionseffekt erzielt werden kann.

Aus der WO 01/13989 A1 ist ein Elektroperforationsverfahren bekannt, bei dem zunächst Hautgewebe gezielt ablatiert wird, um die Haut zu perforieren und auschließend Wirkstoffe einzubringen. Beim Ablatieren kann zur Schmerzlinderung eine Kühlung erfolgen.

Dokument US-A-6 148 232 offenbart eine Vorrichtung wie sie im Oberbegriff von Anspruch 1 beschrieben ist.

Der vorliegenden Erfindung liegt die Aufgabe zugrunde, ein Iontophorese-Elektrodensystem bereitzustellen, welches die zuvor beschriebenen Nachteile beseitigt und eine verbesserte Iontophorese ermöglicht. Diese Aufgabe wird erfindungsgemäß durch ein Iontophorese-Kaltkontaktelektrodensystem mit den Merkmalen des Anspruchs 1 gelöst. Die Unteransprüche definieren jeweils vorteilhafte und bevorzugte Ausführungsformen der Erfindung.

Bisher noch nicht bekannt war ein Iontophorese-Elektrodensystem, das gleichzeitige Iontophorese und Hautkühlung vereint. Die vorliegende Erfindung stellt eine bewegliche Iontophorese-Kaltkontaktelektrode bereit, welche mit einfachen Mitteln die Iontophorese ermöglicht und gleichzeitig die Haut kühlt. Wobei insbesondere der leichte Wechsel des mit einer abrasiven Oberfläche versehenen Wirkstoffträgers, genannt Wirkstoff-Pad, die feste Fixierung des Wirkstoff-Pad auf der Elektrodenfläche, die sehr gute Reinigungsmöglichkeit der Elektrode von Wirkstoffresten nach der Anwendung und insgesamt die unproblematische Handhabung in der Anwendung gewährleistet ist. Die vorliegende Iontophorese-Kaltkontaktelektrode ist darüber hinaus insbesondere in der kosmetischen Anwendung durch die besondere Elektrodenform für die verschiedenen schwierigen Hautbereiche im Gesicht geeignet, und vor allem auch für den Anwender bei der Behandlung leicht anwendbar.

Das erfindungsgemäße Iontophorese-Kaltkontaktelektrodensystem umfasst einen Elektrodenkörper mit einer innen gekühlten und derart nach außen erhabenen, gewölbten elektrisch leitenden Elektodenoberfläche, daß durch diese erhabene, gewölbte Form auch die engen Gesichtspartien um Nase, Mund, Augen und Ohren gut erreicht werden können. Bei Bedarf kann das Kühlelement im Inneren des Elektrodenkörpers durch Umschalten am Steuergerät auch als Heizelement zum Wiedererwärmen der Haut auf die normale Körpertemperatur verwendet werden. Dieser Kalt/Warm-Effekt dient zusätzlich der Hautkonditionierung.

Des weiteren umfasst das erfindungsgemäße Iontophorese-Kaltkontaktelektrodensystem einen Elektrodendeckel mit einer derartigen mittigen großen Öffnung, daß der Elektrodendeckel mit seinem Deckelsteg den vorher auf die Elektrodenfläche aufgelegten, etwas überstehenden Wirkstoff-Pad beim Aufschrauben auf den Elektrodenkörper so auf der Elektodenfläche ganzflächig festklemmt, daß der Wirkstoff-Pad fest fixiert aus der Deckelöffnung herausragt.

Ebenso umfasst das erfindungsgemäße Iontophorese-Kaltkontaktelektrodensystem einen Wirkstoff-Pad aus einem flexiblen, porösen, an der Oberfläche entsprechend stark aufgerauhten elektrisch nicht leitenden Material, der mit der elektrisch geladenen kosmetischen oder medizinischen Wirkstoffmischung, ähnlich einem vollgesogenen Schwamm, getränkt ist. Vorteilhafterweise wird der Wirkstoff-Pad als Wirkstoff-Einwegpad in luftdichter Verpackung gelagert und erst unmittelbar vor der Anwendung auf die Elektrodenfläche aufgelegt und wie oben beschrieben für die Behandlung fixiert. Die in einem Wirkstoff-Einwegpad enthaltene Wirkstoffmenge ist dabei gerade für eine Behandlung bemessen, so daß nach Ende der Behandlung der aufgebrauchte Pad entsorgt werden kann und die Elektrode für eine spätere neue Behandlung gereinigt werden kann.

### Erläuterung der Figuren:

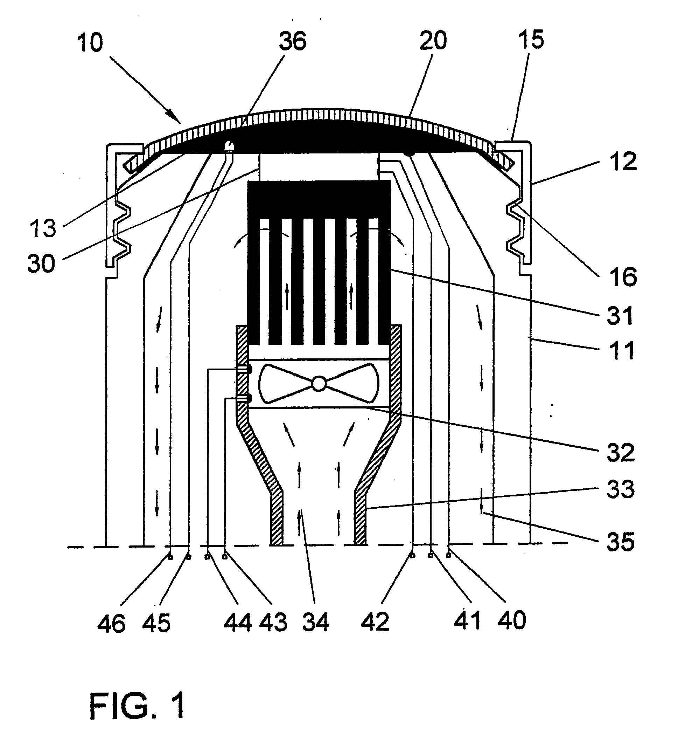

Figur 1 zeigt als Querschnittszeichnung beispielhaft ein erfindungsgemäßes Iontophorese-Kaltkontaktelektrodensystem (10), bestehend aus dem Elektrodenkörper (11) mit der nach oben gewölbten elektrisch leitenden Elektrodenoberfläche (13) und dem aufgesetzten Wirkstoff-Pad (20), welches durch den Deckelsteg (15) des oben offenen Elektrodendeckels (12) auf die nach oben gewölbte Elektrodenoberfläche (13) flächig festgeklemmt ist und erhaben über den Elektrodendeckel (12) herausragt. Das festklemmen des Wirkstoff-Pads (20) durch den Deckelsteg (15) wird durch das Festschrauben des Elektrodendeckels (12) mit seinem Innengewinde (16) auf das Außengewinde des Elektrodenkörpers (11) erreicht. Der rückseitige Abschluß des Elektrodenkörpers ist symbolisch als Strichlinie dargestellt.

Die Elektrodenoberfläche (13) wird mit einem, an ihrer Innenseite angebrachten elektronischen Kühl/Heizelement, vorzugsweise einem handelsüblichen Peltier-Element (30), definiert gekühlt bzw. geheizt, wenn dies zur besseren Konditionierung der Haut für das Einbringen der iontophoretischen Wirkstoffe vorteilhaft ist.

Bei Kühlfunktion erkaltet das Peltierelement (30) an der Elektrodenseite und nimmt so die Wärme von der Elektrodenoberfläche (13) auf und gibt sie auf seiner anderen Seite an einen dort angebrachten Kühlkörper (31) ab, dessen Kühlrippen durch ein Luftkühlsystem gekühlt werden. Bei diesem Luftkühlsystem saugt der vor dem Kühlkörper (31) angeordnete Ventilator (32) die kühle Außenluft (34) durch die Luftleithutze (33) an und bläst durch die Kühlrippen des Kühlkörpers (31) hindurch die erwärmte Abluft (35) durch den Innenraum des Elektrodenkörpers (11) nach Außen ab.

Bei Heizfunktion wird die Stromrichtung im Peltierelement (30) umgepolt, dabei erwärmt sich das Peltierelement an der Elektrodenseite und erwärmt so die Elektrodenoberfläche (13). Das Luftkühlsystem kann hierbei zur Temperaturregelung mit verwendet werden.

Die Temperatur der Elektrodenoberfläche (13) und damit auch die des aufgeklemmten Wirkstoffpads (20) wird durch den in die Elektrodenoberfläche (13) montierten Temperatursensor (36) ermittelt und als Temperatur-Istwert einem Temperaturregler in dem Iontophorese-Steuergerät zugeführt, welcher die gewünschte Kühl- bzw. Heiztemperatur exakt regelt. Die Leitungen (40)- (46) stellen die Anschlußleitungen der elektrischen Komponenten des Kaltkontakt-Elektrodensystems mit dem Iontophorese-Steuergerät dar, wobei Leitung (40) an der Elektrodenoberfläche (13) angeschlossen ist und den Iontophoresestrom führt, die Leitungen (41-42) leiten den Strom zum Peltierelement (30), die Leitungen (43-44) versorgen den Ventilator (32) und die Leitungen (45-46) stellen die Verbindung zu dem Temperatursensor (36) her.

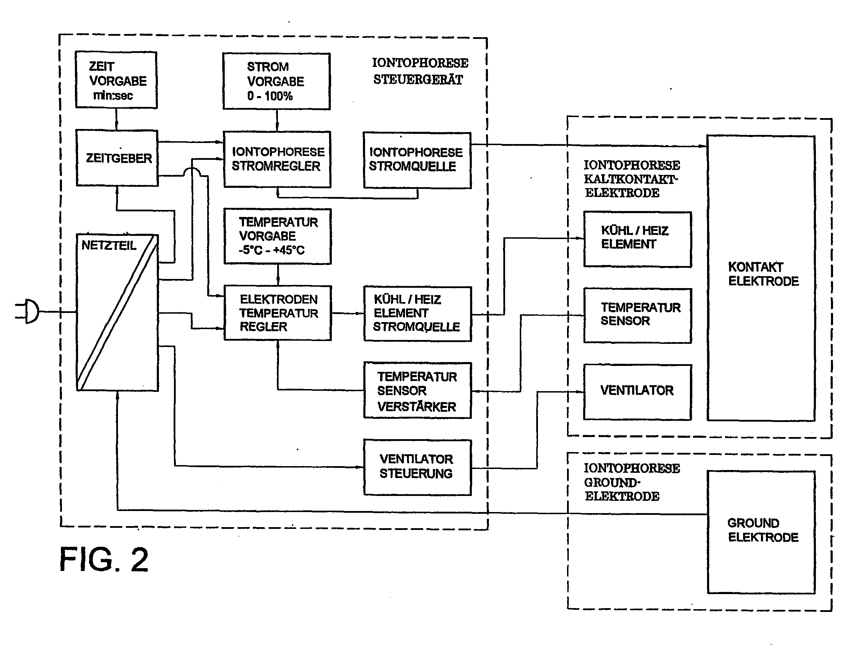

Figur 2 zeigt als Blockschaltplan beispielhaft einen Aufbau eines Iontophoresegerätes mit einem erfindungsgemäßen lontophorese-Kaltkontaktelektrodensystem, der Iontophorese-Groundelektrode und dem lontophorese-Steuergerät. Der Iontophoresestrom wird im Steuergerät durch die Stromvorgabe in der Intensität und durch die Zeitvorgabe im zeitlichen Verlauf durch den Iontophoresestromregler exakt geregelt und fließt, getrieben durch die lontophoresestromquelle, in die Oberfläche der Iontophorese-Kaltkontaktelektrode, weiter durch den aufgeklemmten Iontophorese-Wirkstoffpad in die Patientenhautpartie und weiter durch den Patientenkörper zu der an einer anderen Körperstelle kontaktierten Groundelektrode und dann zurück zum Netzteil im Steuergerät.

Die Kühlung bzw. Heizung der Oberfläche der Iontophorese-Kaltkontaktelektrode wird durch ein Kühl-Heizelement ermöglicht, welches an einen Temperaturregelkreis, bestehend aus Temperaturvorgabe, Elektrodentemperaturregler, umpolbarer Stromquelle und einem an der Kontaktelektrode montierten Temperatursensor, angeschlossen ist. Der für die Luftkühlung des Kühlkörpers erforderliche Ventilator wird durch die Ventilatorsteuerung im Steuergerät angesteuert.

Zur Kühlung/Erwärmung der Elektrodenoberfläche 13 kann auch ein daran vorbei geleitetes und von einem entsprechenden Leitungssystem bereitgestelltes gekühltes/erwärmtes und vorzugsweise flüssiges Mittel, insbesondere ein Gas, verwendet werden.

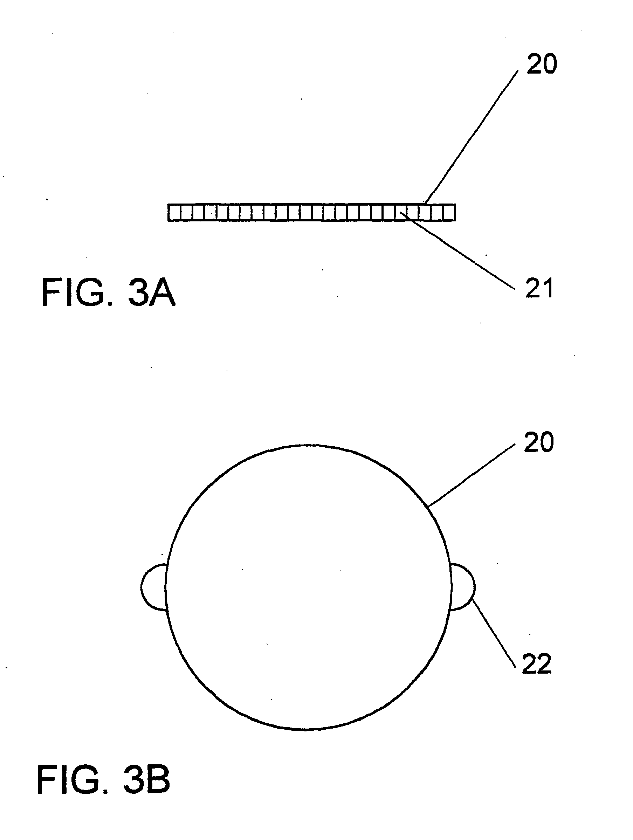

Figur 3A zeigt als Querschnittszeichnung beispielhaft einen mit dem iontophoretischen Wirkstoff (21) getränkten Wirkstoff-Einwegpad (20) aus einem saugfähigen porösen an der Oberfläche für die gewünschte Hautabrasion entsprechend stark aufgerauhten Material. Das Wirkstoff-Einwegpad (20) sollte wegen der besseren Haltbarkeit und der einfachen Anwendung in Einfach-Blister luftdicht verpackt sein, und als Einweg-Pad nach einer Behandlung entsorgt werden.

Figur 3B zeigt ein Wirkstoff-Einwegpad (20) beispielhaft mit zusätzlichen Ansätzen (22) zur leichteren Entnahme aus der Verpackung.

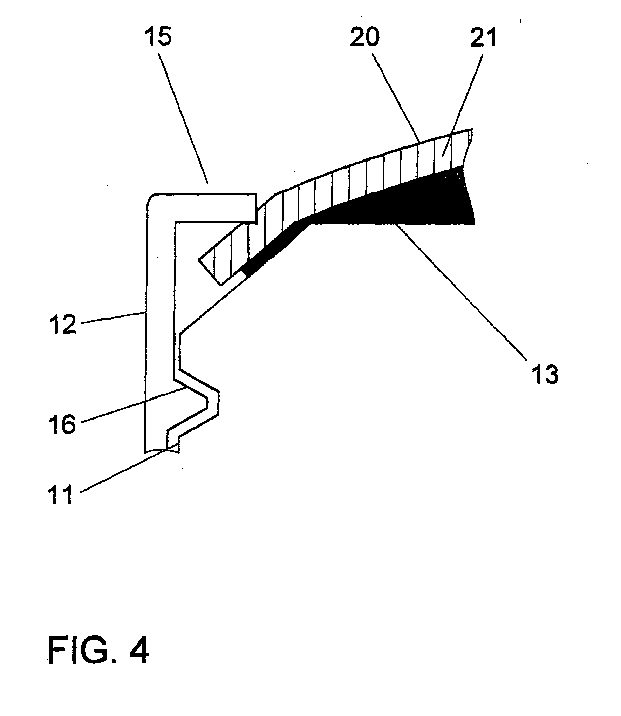

Figur 4 zeigt eine Querschnittszeichnung vergrößert als Detail aus Figur1, wie der festgeschraubte Elektrodendeckel (12) mit seinem Deckelsteg (15) das mit dem iontophoretischen Wirkstoff (21) getränkte Wirkstoff-Pad (20) am Rand einklemmt und auf die nach oben gewölbte elektrisch leitende Elektrodenoberfläche (13) ganzflächig festklemmt und wie das Wirkstoff-Pad (20) über den Elektrodendeckel (12) herausragt, so daß bei der Iontophoresebehandlung nur das Wirkstoff-Pad (20) die zu behandelnde Hautpartie des Patienten zur Wirkstoffübertragung berührt.

## Patentansprüche

1. Iontophorese-Kaltkontaktelektrodensystem,
mit einem Elektrodenkörper (11) mit einer definiert gekühlten Elektrodenoberfläche (13) zum Einbringen von iontophoretischen Wirkstoffen in die menschliche Haut und zum gleichzeitigen Kühlen der menschlichen Haut, um die elektrische Leitfähigkeit der Haut und damit das Einbringen der iontophoretischen Wirkstoffe in die Haut zu verbessern,
**gekennzeichnet durch**
eine Klemmvorrichtung (12, 15) zum Fixieren von Wirkstoff-Pads (20), welche mit den iontophoretischen Wirkstoffen getränkt sind, auf der Elektrodenoberfläche (13).

2. Iontophorese-Kaltkontaktelektrodensystem nach Anspruch 1,
**dadurch gekennzeichnet,**
**daß** die Kühltemperatur der Elektrodenoberfläche (13) durch einen integrierten Temperatursensor (36) erfasst und über eine Regelung auf eine Temperatur bis -5 (minus fünf) Grad Celsius definiert wählbar gesteuert wird.

3. Iontophorese-Kaltkontaktelektrodensystem nach Anspruch 2,
**dadurch gekennzeichnet,**
**daß** die kühlbare Elektrodenoberfläche (13) durch ein Peltier-Element (30) definiert gekühlt wird.

4. Iontophorese-Kaltkontaktelektrodensystem nach Anspruch 2,
**dadurch gekennzeichnet,**
**daß** die kühlbare Elektrodenoberfläche (13) an ein Leitungssystem angeschlossen ist und diese durch das Vorbeileiten eines entsprechend gekühlten, flüssigen Kühlmittels gekühlt wird.

5. Iontophorese-Kaltkontaktelektrodensystem nach Anspruch 4,
**dadurch gekennzeichnet,**
**daß** das Kühlmittel ein Gas ist.

6. Iontophorese-Kaltkontaktelektrodensystem nach einem der vorhergehenden Ansprüche,
**dadurch gekennzeichnet,**
**daß** dieselbe Elektrodenoberfläche (13), die dann auf die menschliche Haut aufgesetzt wird, bei Bedarf auch definiert bis vorzugsweise auf +45 Grad Celsius zu erwärmen ist und so die menschliche Haut bei entsprechender Notwendigkeit auch wieder aufheizt.

7. Iontophorese-Kalkontaktelektrodensystem nach Anspruch 3 und 6,
**dadurch gekennzeichnet,**
**daß** die Erwärmung der Elektrodenoberfläche (13) über das Umschalten der Polarität am Peltier-Element (30) erfolgt.

8. Iontophorese-Kaltkontaktelektrodensystem nach Anspruch 6,
**dadurch gekennzeichnet,**
**daß** die Elektrodenoberfläche (13) an ein Leitungssystem angeschlossen ist und diese durch das Vorbeileiten eines entsprechend erwärmten Mittels erwärmt wird.

9. Iontophorese-Kaltkontaktelektrodensystem nach Anspruch 8,
**dadurch gekennzeichnet,**
**daß** das zur Erwärmung der Elektrodenoberfläche (13) benutzte Mittel ein Gas ist.

10. Iontophorese-Kaltkontaktelektrodensystem nach einem der vorhergehenden Ansprüche,
**dadurch gekennzeichnet,**
**daß** die Klemmvorrichtung derart ausgeführt ist, daß auf dem Elektrodenkörper (11), mit einer nach außen gewölbten, elektrisch leitenden Elektrodenoberfläche (13), der aus einem porösen, nicht leitenden Material bestehende und mit dem iontophoretischem Wirkstoff getränkte Wirkstoff-Pad (20) durch einen Deckelsteg (15) eines oben offenen Elektrodendeckels (12) ganzflächig so auf der Elektrodenoberfläche (13) festgeklemmt wird, dass der Wirkstoff-Pad erhaben über den Elektrodenkörper herausragend fixiert wird und dadurch über die menschliche Haut geführt werden kann und einen lontophorese-Stromkreis zur einer an einem anderen Körperbereich angeschlossenen Groundelektrode schließt.

11. Iontophorese-Kaltkontaktelektrodensystem nach Anspruch 10,
**dadurch gekennzeichnet,**
**dass** die Klemmvorrichtung derart ausgeführt ist, dass der Elektrodendeckel (12) mit einem Innengewinde (16) auf ein Außengewinde des Elektrodenkörpers (11) festgeschraubt wird.

12. Iontophorese-Kaltkontaktelektrodensystem nach Anspruch 10,
**dadurch gekennzeichnet,**
**dass** die Klemmvorrichtung derart ausgeführt ist, dass der Elektrodendeckel (12) mit einem Bajonettverschluss auf dem Elektrodenkörper (11) fixiert wird.

13. Iontophorese-Kaltkontaktelektrodensystem nach Anspruch 10,
**dadurch gekennzeichnet,**
**dass** die Klemmvorrichtung derart ausgeführt ist, dass der Elektrodendeckel (12) mit einer Einrastnocke auf dem Elektrodenkörper (11) fixiert wird.

14. Iontophorese-Kaltkontaktelektrodensystem nach Anspruch 10,
**dadurch gekennzeichnet,**
**dass** die Klemmvorrichtung derart ausgeführt ist, dass der Elektrodendeckel (12) durch einen Preßsitz auf den Elektrodenkörper (11) gepresst und dadurch fixiert wird.

15. Iontophorese-Kaltkontaktelektrodensystem nach einem der Ansprüche 9-14,
**dadurch gekennzeichnet,**
**dass** der Wirkstoff-Pad (20) aus einem flexiblen, porösen, an der Oberfläche aufgerauhten, nichtleitenden Material besteht und mit einem oder mehreren elektrisch leitenden, in Flüssigkeit oder Gel oder Creme gelösten iontophoretischen Wirkstoffen getränkt ist.

16. Iontophorese-Kaltkontaktelektrodensystem nach Anspruch 9-15,
**dadurch gekennzeichnet,**
**dass** der Wirkstoff-Pad (20) mit medizinischen Wirkstoffen getränkt ist , die für das Einbringen in die menschliche Haut bestimmt sind.

17. Iontophorese-Kaltkontaktelektrodensystem nach einem der Ansprüche 9-16,
**dadurch gekennzeichnet,**
**daß** der Wirkstoff-Pad (20) mit kosmetischen Wirkstoffen getränkt ist, die für das Einbringen in die menschliche Haut bestimmt sind.

18. Iontophorese-Kaltkontaktelektrodensystem nach einem der Ansprüche 9-17,
**dadurch gekennzeichnet,**
**daß** der Wirkstoff-Pad (20) mit nahrungsergänzenden Wirkstoffen getränkt ist , die für das Einbringen in die menschliche Haut bestimmt sind.

19. Iontophorese-Kaltkontaktelektrodensystem nach einem der Ansprüche 9-18,
**dadurch gekennzeichnet,**
**daß** der Wirkstoff-Pad (20) in Kreisform ausgeführt ist.

20. Iontophorese-Kaltkontaktelektrodensystem nach einem der Ansprüche 9-19,
**dadurch gekennzeichnet,**
**dass** der Wirkstoff-Pad (20) als Vieleck ausgeführt ist.

21. Iontophorese-Kaltkontaktelektrodensystem nach einem der Ansprüche 9-20,
**dadurch gekennzeichnet,**
**daß** der Wirkstoff-Pad (20) seitlich angebrachte Griff-Ansätze (22) hat, die zur besseren Entnahme dienen.

22. Iontophorese-Kaltkontaktelektrodensystem nach einem der Ansprüche 9-21,
**dadurch gekennzeichnet,**
**daß** der Wirkstoff-Pad (20) ein Wirkstoff-Einwegpad ist.

23. Iontophorese-Kaltkontaktelektrodensystem nach einem der Ansprüche 1-22,
**dadurch gekennzeichnet,**
**daß** die Elektrodenoberfläche (13) zum Zwecke der Abrasion von verhornten Zellen der oberen Hautschicht gleichzeitig zum Kühlen und Einbringen der iontophoretischen Wirkstoffe in die Haut ausgestaltet ist.

## Claims

1. Iontophoresis cold-contact electrode system,
having an electrode body (11) with a defined cooled electrode surface (13) for the introduction of iontophoretic active substances into the human skin and for the simultaneous cooling of the human skin in order to improve the electrical conductivity of the skin and therewith the introduction of the iontophoretic active substances into the skin,
**characterized by**
a clamping device (12, 15) for fixing active-substance pads (20) impregnated with intophoretic active substances to the electrode surface (13).

2. Iontophoresis cold-contact electrode system according to Claim 1,
**characterized in that**
the cooled temperature of the electrode surface (13) is determined by an integrated temperature sensor (36) and is controlled by regulation to a temperature of down to -5 (minus five) degrees Celsius in a defined and selectable way.

3. Iontophoresis cold-contact electrode system according to Claim 2,
**characterized in that**
the coolable electrode surface (13) is cooled in a defined way by a Peltier element (30).

4. Iontophoresis cold-contact electrode system according to Claim 2,
**characterized in that**
the coolable electrode surface (13) is connected to a conduct system and it is cooled by conveying a suitably cooled, fluid coolant past it.

5. Iontophoresis cold-contact electrode system according to Claim 4,
**characterized in that**
the coolant is a gas.

6. Iontophoresis cold-contact electrode system according to any one of the preceding claims,
**characterized in that**
the same electrode surface (13) that is then applied to the human skin can, if necessary, also be heated in a defined way preferably to +45 degrees Celsius and thus also heats the human skin again if there is a corresponding need.

7. Iontophoresis cold-contact electrode system according to Claims 3 and 6,
**characterized in that**
the heating of the electrode surface (13) takes place by switching the polarity at the Peltier element (30).

8. Iontophoresis cold-contact electrode system according to Claim 6,
**characterized in that**
the electrode surface (13) is connected to a conduct system and it is heated by conveying a suitably heated medium past it.

9. Iontophoresis cold-contact electrode system according to Claim 8,
**characterized in that**
the medium used to heat the electrode surface (13) is a gas.

10. Iontophoresis cold-contact electrode system according to any one of the preceding claims,
**characterized in that**
the clamping device is constructed in such a way that the active-substance pad (20) composed of a porous, nonconducting material and impregnated with the iontophoretic active substance is firmly clamped onto the electrode body (11) having an outwardly arched, electrically conducting electrode surface (13) by a cover flange (15) of an upwardly open electrode cover (12) over the entire area onto the electrode surface (13) in such a way that the active-substance pad is fixed so as to project in a raised manner beyond the electrode body and can thereby be conveyed over the human skin and closes an iontophoresis current circuit to a ground electrode connected to another body region.

11. Iontophoresis cold-contact electrode system according to Claim 10,
**characterized in that**
the clamping device is constructed in such a way that the electrode cover (12) is firmly screwed onto an external thread of the electrode body (11) by means of an internal thread (16).

12. Iontophoresis cold-contact electrode system according to Claim 10,
**characterized in that**
the clamping device is constructed in such a way that the electrode cover (12) is fixed by a bayonet closure to the electrode body (11).

13. Iontophoresis cold-contact electrode system according to Claim 10,
**characterized in that**
the clamping device is constructed in such a way that the electrode cover (12) is fixed to the electrode body (11) by a latching cam.

14. Iontophoresis cold-contact electrode system according to Claim 10,
**characterized in that**
the clamping device is constructed in such a way that the electrode cover (12) is pressed onto and thereby fixed to the electrode body (11) by a press seating.

15. Iontophoresis cold-contact electrode system according to any one of Claims 9-14,
**characterized in that**
the active-substance pad (20) is composed of a flexible, porous nonconducting material roughened on the surface area and is impregnated with one or more electrically conducting iontophoretic active substances dissolved in liquid or gel or cream.

16. Iontophoresis cold-contact electrode system according to Claims 9-15,
**characterized in that**
the active-substance pad (20) is impregnated with medicinally active substances that are intended for introduction into the human skin.

17. Iontophoresis cold-contact electrode system according to any one of Claims 9-16,
**characterized in that**
the active-substance pad (20) is impregnated with cosmetic active substances that are intended for introduction into the human skin.

18. Iontophoresis cold-contact electrode system according to any one of Claims 9-17,
**characterized in that**
the active-substance pad (20) is impregnated with nutrition-supplementing active substances that are intended for introduction into the human skin.

19. Iontophoresis cold-contact electrode system according to any one of Claims 9-18,
**characterized in that**
the active-substance pad (20) is constructed in circular shape.

20. Iontophoresis cold-contact electrode system according to any one of Claims 9-19,
**characterized in that**
the active-substance pad (20) is constructed as a polygon.

21. Iontophoresis cold-contact electrode system according to any one of Claims 9-20,
**characterized in that**
the active-substance pad (20) has laterally fitted gripping extensions (22) that serve for better removal.

22. Iontophoresis cold-contact electrode system according to any one of Claims 9-21,
**characterized in that**
the active-substance pad (20) is a disposable active-substance pad.

23. Iontophoresis cold-contact electrode system according to any one Claims 1-22,
**characterized in that**
the electrode surface (13) is constructed for the simultaneous cooling and introduction of the iontophoretic active substances into the skin for the purpose of abrasion of horny cells of the upper skin layer.

## Revendications

1. Système d'électrode iontophorétique à contact à froid,
avec un corps d'électrode (11) avec une surface d'électrode (13) refroidie de façon définie pour l'introduction de substances actives iontophorétiques dans la peau humaine, et pour le refroidissement simultané de la peau humaine, pour améliorer la conductibilité électrique de la peau et avec elle l'introduction de substances actives iontophorétiques dans la peau,
**caractérisé par**
un dispositif de serrage (12, 15) pour fixer des tampons de substances actives (20), qui sont imprégnés de substances actives iontophorétiques, à la surface de l'électrode (13).

2. Système d'électrode iontophorétique à contact à froid selon la revendication 1,
**caractérisé en ce que**
la température de refroidissement de la surface d'électrode (13) est détectée par un capteur de température (36) intégré, et est commandée précisément de façon sélectionnable par un dispositif de régulation à une température allant jusqu'à -5 (moins cinq) degrés Celsius.

3. Système d'électrode iontophorétique à contact à froid selon la revendication 2,
**caractérisé en ce que**
la surface d'électrode (13) pouvant être refroidie est refroidie de façon définie par un élément Peltier (30).

4. Système d'électrode iontophorétique à contact à froid selon la revendication 2,
**caractérisé en ce que**
la surface d'électrode (13) pouvant être refroidie est raccordée à un système de conduite, et celle-ci est refroidie par le passage d'un réfrigérant fluide refroidi en conséquence.

5. Système d'électrode iontophorétique à contact à froid selon la revendication 4,
**caractérisé en ce que**,
le réfrigérant est un gaz.

6. Système d'électrode iontophorétique à contact à froid selon une des revendications précédentes,
**caractérisé en ce que**
la même surface d'électrode (13), qui est appliquée sur la peau humaine, peut être chauffée si besoin et de façon définie jusqu'à de préférence +45 degrés Celsius, et elle réchauffe ainsi la peau humaine selon les besoins correspondants.

7. Système d'électrode iontophorétique à contact à froid selon les revendications 3 et 6,
**caractérisé en ce que**,
le réchauffement de la surface d'électrode (13) a lieu par commutation de la polarité sur l'élément Peltier (30).

8. Système d'électrode iontophorétique à contact à froid selon la revendication 6,
**caractérisé en ce que**,
la surface d'électrode (13) est raccordée à un système de conduite, et celle-ci est chauffée par le passage d'un milieu chauffé en conséquence.

9. Système d'électrode iontophorétique à contact à froid selon la revendication 8,
**caractérisé en ce que**,
le milieu utilisé pour chauffer la surface d'électrode (13) est un gaz.

10. Système d'électrode iontophorétique à contact à froid selon une des revendications précédentes,
**caractérisé en ce que**
le dispositif de serrage est conçu de telle sorte que le tampon de substance active (20) formé de matériau poreux non conducteur et imprégné de substance active iontophorétique est bloqué sur le corps d'électrode (11), avec une surface d'électrode électriquement conductrice incurvée vers l'extérieur, par une bride de couvercle (15) d'un couvercle d'électrode (12) ouvert dans la partie supérieure sur toute la surface à la surface d'électrode (13), de sorte que le tampon de substance active est fixé en dépassant en relief du corps d'électrode, et peut être guidé sur la peau humaine, et ferme un circuit iontophorétique vers une électrode de terre raccordée à une autre zone corporelle.

11. Système d'électrode iontophorétique à contact à froid selon la revendication 10,
**caractérisé en ce que**
le dispositif de serrage est conçu de telle sorte que le couvercle d'électrode (12) est vissé avec un filet interne (16) sur un filet externe du corps d'électrode (11).

12. Système d'électrode iontophorétique à contact à froid selon la revendication 10,
**caractérisé en ce que**
le dispositif de serrage est conçu de telle sorte que le couvercle d'électrode (12) est fixé avec une fermeture à baïonnette sur le corps d'électrode (11).

13. Système d'électrode iontophorétique à contact à froid selon la revendication 10,
**caractérisé en ce que**
le dispositif de serrage est conçu de telle sorte que le couvercle d'électrode (12) est fixé avec une came d'enclenchement sur le corps d'électrode (11).

14. Système d'électrode iontophorétique à contact à froid selon la revendication 10,
**caractérisé en ce que**
le dispositif de serrage est conçu de telle sorte que le couvercle d'électrode (12) est serré par un ajustement serré sur le corps d'électrode (11) et est fixé à celui-ci.

15. Système d'électrode iontophorétique à contact à froid selon une des revendications 9 à 14,
**caractérisé en ce que**
le tampon de substance active (20) est formé d'un matériau flexible, poreux, brossé en surface et non conducteur, et il est imprégné d'une ou de plusieurs substances actives iontophorétiques dissoutes sous forme de liquide, de gel ou de crème, électriquement conductrices.

16. Système d'électrode iontophorétique à contact à froid selon une des revendications 9 à 15,
**caractérisé en ce que**
le tampon de substance active (20) est imprégné de substances actives médicamenteuses qui sont prévues pour l'introduction dans la peau humaine.

17. Système d'électrode iontophorétique à contact à froid selon une des revendications 9 à 16,
**caractérisé en ce que**
le tampon de substance active (20) est imprégné de substances actives cosmétiques qui sont prévues pour l'introduction dans la peau humaine.

18. Système d'électrode iontophorétique à contact à froid selon une des revendications 9 à 17,
**caractérisé en ce que**
le tampon de substance active (20) est imprégné de substances actives de compléments alimentaires qui sont prévues pour l'introduction dans la peau humaine.

19. Système d'électrode iontophorétique à contact à froid selon une des revendications 9 à 18,
**caractérisé en ce que**
le tampon de substance active (20) est réalisé sous forme circulaire.

20. Système d'électrode iontophorétique à contact à froid selon une des revendications 9 à 19,
**caractérisé en ce que**
le tampon de substance active (20) est réalisé comme un polygone.

21. Système d'électrode iontophorétique à contact à froid selon une des revendications 9 à 20,
**caractérisé en ce que**
le tampon de substance active (20) comprend des saillies de préhension (22) appliquées latéralement qui favorisent le retrait.

22. Système d'électrode iontophorétique à contact à froid selon une des revendications 9 à 21,
**caractérisé en ce que**
le tampon de substance active (20) est un tampon de substances actives à usage unique.

23. Système d'électrode iontophorétique à contact à froid selon une des revendications 1 à 22,
**caractérisé en ce que**
la surface d'électrode (13) est conçue à des fins d'abrasion des cellules calleuses de la couche cutanée supérieure simultanément au refroidissement et à l'introduction des substances actives iontophorétiques dans la peau.
